# EUROPEAN PATENT SPECIFICATION

(11) **EP 3 641 797 B1**
(45) Date of publication and mention of the grant of the patent: **25.11.2026**
(21) Application number: 18821177.5
(22) Date of filing: 21.06.2018
(51) Int. Cl.: A61K 36/185, A61K 31/7028, C07C 62/06, A61P 31/12, A61P 35/00, C07D 311/60, C07H 15/24

(54) **PROCESS FOR THE PREPARATION OF STANDARDIZED COMPOSITION OF ARJUNOGLUCOSIDE FROM THE BARK OF TERMINALIA ARJUNA**
VERFAHREN ZUR HERSTELLUNG EINER STANDARDISIERTEN ZUSAMMENSETZUNG VON ARJUNOGLUKOSID AUS DER RINDE VON TERMINALIA ARJUNA
PROCÉDÉ DE PRÉPARATION D'UNE COMPOSITION NORMALISÉE D'ARJUNOGLUCOSIDE PROVENANT DE L'ÉCORCE DE TERMINALIA ARJUNA

(30) Priority: 21.06.2017 US 201762522801 P
(43) Date of publication of application: 29.04.2020
(73) Proprietor: Sami-Sabinsa Group Limited, Bangalore, Karnataka 560058 (IN)
(72) Inventor: MAJEED, Muhammed, East Windsor, NJ 08520 (US); NAGABHUSHANAM, Kalyanam, East Windsor, NJ 08520 (US); BHAT, Beena, Bangalore 560058 (IN)
(74) Representative: Dr. Gassner & Partner mbB
(86) International application number: PCT/US2018/038738
(87) International publication number: WO 2018/237135

(56) References cited:
- US-A- 5 916 919
- US-A1- 2007 122 506
- US-A1- 2007 122 506
- HONDA T ET AL: "ARJUNGENIN, ARJUNGLUCOSIDE I, AND ARJUNGLUCOSIDE II. A NEW TRITERPENE AND NEW TRITERPENE GLUCOSIDES FROM TERMINALIA ARJUNA", BULLETIN OF THE CHEMICAL SOCIETY OF JAPAN, CHEMICAL SOCIETY OF JAPAN, TOKYO, JP, vol. 49, no. 11, 1 January 1986 (1986-01-01), pages 3213 - 3218, XP008067925, ISSN: 0009-2673
- ANUMITA SAHA ET AL: "Characterisation of polyphenols in Terminalia arjuna bark extract", INDIAN JOURNAL OF PHARMACEUTICAL SCIENCES, vol. 74, no. 4, 1 July 2012 (2012-07-01), IN, pages 339 - 347, XP055569934, ISSN: 0250-474X, DOI: 10.4103/0250-474X.107067
- SUBASH CHANDRA VERMA ET AL: "Microwave extraction and rapid isolation uf arjunic acid from Terminalia arjuna (Roxb. ex DC.) stem bark and quantification of arjunic acid and arjunolic acid using HPLC-PDA technique", JOURNAL OF SEPARATION SCIENCE., vol. 35, no. 13, 1 July 2012 (2012-07-01), DE, pages 1627 - 1633, XP055569938, ISSN: 1615-9306, DOI: 10.1002/jssc.201200083
- TSUYUKI TAKAHIKO ET AL: "A new triterpene glucoside from Terminalia arjuna. Arjunoglucoside III", BULLETIN OF THE CHEMICAL SOCIETY OF JAPAN, CHEMICAL SOCIETY OF JAPAN, TOKYO, JP, vol. 52, no. 10, 1 January 1979 (1979-01-01), pages 3127 - 3128, XP009517914, ISSN: 0009-2673, DOI: 10.1246/BCSJ.52.3127
- SAXENA MOHIT ET AL: "Cytotoxic agents from Terminalia arjuna; including Supporting Information", PLANTA MEDICA, THIEME VERLAG, DE, vol. 73, no. 14, 31 October 2007 (2007-10-31), pages 1486 - 1490, XP009525225, ISSN: 0032-0943, [retrieved on 20071116], DOI: 10.1055/S-2007-990258
- PAARAKH ET AL: "Terminalia arjuna (Roxb.) Wt. and Arn.: a review.", INTERNATIONAL JOURNAL OF PHARMACOLOGY 6.5, vol. 6, no. 5, 1 January 2010 (2010-01-01), pages 515 - 534, XP055824208, Retrieved from the Internet <URL:https://docsdrive.com/pdfs/ansinet/ijp/2010/515-534.pdf>
- HONDA T. ET AL.: "Arjungenin, Arjunglucoside I, and Arjunglucoside II. A New Triterpene and New Triterpene Glucosides from Terminalia arjuna", BULLETIN OF THE CHEMICAL SOCIETY OF JAPAN, vol. 49, no. 11, 1976, pages 3213 - 3218, XP008067925
- SAHA A. ET AL.: "Characterization of polyphenols in Terminalia arjuna bark extract", INDIAN JOURNAL OF PHARMACEUTICAL SCIENCES, vol. 74, no. 4, 2012, pages 339 - 347, XP055569934
- VERMA S. C. ET AL.: "Microwave extraction and rapid isolation uf arjunic acid from Terminalia arjuna (Roxb. ex DC.) stem bark and quantification of arjunic acid and arjunolic acid using HPLC-PDA technique", JOURNAL OF SEPARATION SCIENCE, vol. 35, no. 13, 2012, pages 1627 - 1633, XP055569938
- TSUYUKI T. ET AL.: "A new triterpene glucoside from Terminalia arjuna. Arjunoglucoside III", BULLETIN OF THE CHEMICAL SOCIETY OF JAPAN, vol. 52, no. 10, 1979, pages 3127 - 3128, XP009517914, DOI: 10.1246/bcsj.52.3127

## Description

### BACKGROUND OF THE INVENTION

### Field of the invention

The present invention discloses a novel process for the isolation of bioactive compounds from bark of *Terminalia arjuna* and a composition comprising arjunoglucosides.

### Description of prior art

*Terminalia arjuna* or arjuna is a well known medicinal plant used in the ancient Ayurvedic medicine. The bark of *Terminalia arjuna* reported to contain many bioactive compounds, which can be tapped for use in the treatment of many diseases. The following prior art describe the important biological effects of *Terminalia arjuna.*
1. Pawar & Bhutani, Effect of oleanane triterpenoids from Terminalia arjuna-a cardioprotective drug on the process of respiratory oxyburst, Phytomedicine, 2005;25:391-393
2. Sandhu et al., Effects of Withania somnifera (Ashwagandha) and Terminalia arjuna (Arjuna) on physical performance and cardiorespiratory endurance in healthy young adults, Int J Ayurveda Res, 2010;1(3): 144-149.
3. Zafar et al., Terminalia arjuna: Alternative Treatment for Cardiovascular Diseases, Int. J. Pharm. Sci. Rev. Res., 2015;35(2): 52-56.
4. Patel et al., Development and Validation of Stability Indicating Assay for Arjuna Caplets and Stability Studies with Concurrent Phytochemical Investigations, Inventi Rapid: Planta Activa, 2015;1:1-5.
5. Chandra Sekhar et al., Terminalia arjuna bark extract attenuates picrotoxin-induced behavioral changes by activation of serotonergic, dopaminergic, GABAergic and antioxidant systems, Chinese Journal of Natural Medicines, 2017, 15(8): 584-596

Arjunoglucoside, a compound obtained from bark of *Terminalia arjuna* is well documented in literatures for its cardio-protective activity. Other than its cardioprotective property, *Terminalia arjuna* and its bark extract reported for wide range of activities such as anticancer, hepatoprotective, antiviral, antioxidant, anti-asthmatic, anti-fertility, anti-diabetic, wound healing, anti-platelet and anticoagulant, anti-bacterial and anti-fungal activity. (Saxena et al., Cytotoxic agents from Terminalia arjuna, Planta Med. 2007;73(14):1486-90).

There are many processes disclosed in the literature for the isolation of Arjunoglucoside and its derivatives from bark of *Terminalia arjuna* and other species of *Terminalia.* Anbalangan et al. (A. Anbalagan, Phytochemical and pharmacological studies on Terminalia arjuna (Roxb.) weight & Arnot and Borreria Hispida (Linn.) K. Schum, Ph. D Thesis, http://shodhganga.inflibnet.ac.in/bitstream/10603/4547/8/08_chapter%202.pdf (Accessed on 13 June 2018) reported a methanolic extract of stem bark of *Terminalia arjuna* chromatographed over silica gel benzene and eluted with solvents of increasing polarity viz., benzene, chloroform and methanol. Total 6 compounds were identified from methanolic extract of stem bark of *Terminalia arjuna* such as 3-oxo-olean-12-ene-28-oic acid, Methyl maslinate, Hederagenin Methyl ester, Hederagenin, Maslinic acid and Arjunolic acid. The other processes for the isolation of arjunoglucosides are described in the following prior art documents:
1. Tsuyuki et al., A new triterpene compound from Terminalia arjuna. Arjunoglucoside III. Bulletin of the chemical society of Japan, 1979; 52(10); 3127-3128
2. Honda et al., Arjungenin, Arjunglucoside I, and Arjunglucoside II. A New Triterpene and New Triterpene Glucosides from Terminalia arjuna. Bulletin of the Chemical Society of Japan, 1976;49(11):3213-3218. The document discloses the extraction of arjunic acid methyl ester diacetate, arjunolic acid methyl ester, arjungenin, arjunetin hexaacetate, arjunoglucoside I and arjunoglucoside II from the bark of *Terminalia arjuna* by methanol extraction and lead acetate precipitation.
3. Pertuit et al., A New Aromatic Compound from the Stem Bark of Terminalia catappa, Nat Prod Commun. 2015;10(6):1005-1007.
Saha A et al., Characterisation of Polyphenols in Terminalia arjuna Bark Extract. Indian J Pharm Sci. 2012 Jul;74(4):339-47 discloses the characterization of polyphenols in aqueous-methanol extracts (AMEs) and aqueous extracts (AEs) of *Terminalia arjuna* bark.
US 2007/122506 A1 discloses a process for the isolation of oleane compounds from the bark of *Terminalia arjuna,* in particular the isolation of 2-sitosterol, arjunic acid, arjunolic acid, 2-sitosterol-D-glucoside, arjungenin, arjunetin and arjunoglucoside I. The compounds were extracted with a polar organic solvent at a temperature in the range of 20 to 40 °C.
Paarakh, P. M., Terminalia arjuna (Roxb.) Wt. and Arn.: A Review, International Journal of Pharmacology 6 (5), 515-534, 2010 discloses that Terminalia arjuna (Roxb.) Wt. and Arn. (Arjuna; Combretaceae) is a widely used medicinal plant throughout India and popular in various indigenous system of medicine like ayurveda, siddha and unani. The review gives a detailed survey of the literature on pharmacognosy, phytochemistry and pharmacological activities of the plant.

Though the isolation of arjunoglucoside and its derivatives are already reported in the literature, inventors first time reported novel process for the preparation of standardized composition of arjunoglucoside from bark of *Terminalia arjuna.* The novel process for isolation arjunoglucoside and its derivatives is both economically viable and industrially scalable.

The principle objective of the invention is to disclose a novel process for the isolation and preparation of bioactive compounds from bark of *Terminalia arjuna.*

It is another objective of invention to disclose a composition comprising arjunoglucosides.

The present invention fulfils the aforesaid objectives and provides further related advantages.

### SUMMARY OF THE INVENTION

The present invention is as set out in the appended set of claims.

### BRIEF DESCRIPTION OF THE DRAWINGS

**Fig. 1a** shows the proton NMR spectrum for the identification of Arjunic acid isolated from the bark of *Terminalia arjuna.*
**Fig.** 1b shows the carbon NMR spectrum for the identification of Arjunic acid isolated from the bark of *Terminalia arjuna.*
**Fig. 2a** shows the proton NMR spectrum for the identification of Arjunolic acid isolated from the bark of *Terminalia arjuna.*
**Fig. 2b** shows the carbon NMR spectrum for the identification of Arjunolic acid isolated from the bark of *Terminalia arjuna.*
**Fig. 3a** shows the proton NMR spectrum for the identification of Arjungenin isolated from the bark of *Terminalia arjuna.*
**Fig. 3b** shows the carbon NMR spectrum for the identification of Arjungenin isolated from the bark of *Terminalia arjuna.*
**Fig. 4a** shows the proton NMR spectrum for the identification of Arjunoglucoside-I isolated from the bark of *Terminalia arjuna.*
**Fig. 4b** shows the carbon NMR spectrum for the identification of Arjunoglucoside-I isolated from the bark of *Terminalia arjuna.*
**Fig. 5a** shows the proton NMR spectrum for the identification of Arjunoglucoside-II isolated from the bark of *Terminalia arjuna.*
**Fig. 5b** shows the carbon NMR spectrum for the identification of Arjunoglucoside-II isolated from the bark of *Terminalia arjuna.*
**Fig. 6a** shows the HPLC chromotogram for the identification of Arjunoglucoside-II methanolic extract isolated from the bark of *Terminalia arjuna.*
**Fig. 6b** shows the HPLC chromotogram for the identification of enriched Arjunoglucoside-II isolated from the bark of *Terminalia arjuna.*
**Fig. 7a** shows the proton NMR spectrum for the identification of Arjunetin isolated from the bark of *Terminalia arjuna.*
**Fig. 7b** shows the carbon NMR spectrum for the identification of Arjunetin isolated from the bark of *Terminalia arjuna.*
**Fig. 8a** shows the proton NMR spectrum for the identification of catechin isolated from the bark of *Terminalia arjuna.*
**Fig. 8b** shows the carbon NMR spectrum for the identification of catechin isolated from the bark of *Terminalia arjuna.*

### DESCRIPTION OF MOST PREFFERED EMBODIMENTS

In the most preferred embodiment, the invention discloses a novel process for the isolation of bioactive compounds from bark of *Terminalia arjuna,* said process comprising steps of:
(a) Charging *Terminalia arjuna* bark powder into an extractor,
(b) Adding 3 volumes of ethanol or methanol to *Terminalia arjuna* bark powder and refluxing for 3 hours at 65 to 70 °C,
(c) Filtering the ethanol or methanol extract of step b) and concentrating under vacuum to get a brown coloured powder,
(d) Dissolving the powder obtained from step c) into 5 volumes of de-mineralize water and stirring thoroughly for 1 hr at 50-55°C to obtain a solution,
(e) Transferring the solution from step d) to separating funnel and extracting with 2 volumes of solvent, 6 times, and separating the aqueous and organic layer,
(f) Collecting and combining the solvent fractions 1-6 from step e and concentrating under vacuum to obtain brown coloured powder,
(g) Loading the powder of step f) into a silica gel and eluting with chloroform: methanol,
(h) Identifying the compounds from step g) as Arjunic acid represented by STR#1, Arjunolic acid represented by STR#2, Arjungenin represented by STR#3, Arjunoglucoside-I represented by STR#4, Arjunoglucoside-II represented by STR#5, Arjunetin represented by STR#6, and catechin represented by STR#7.

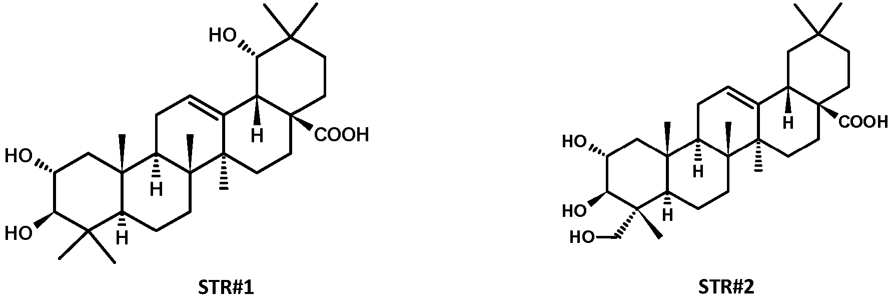

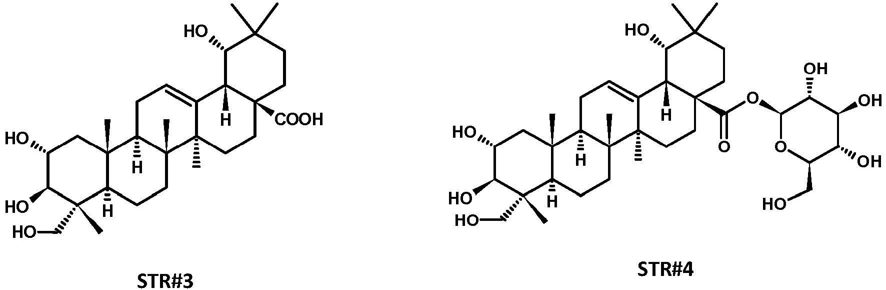

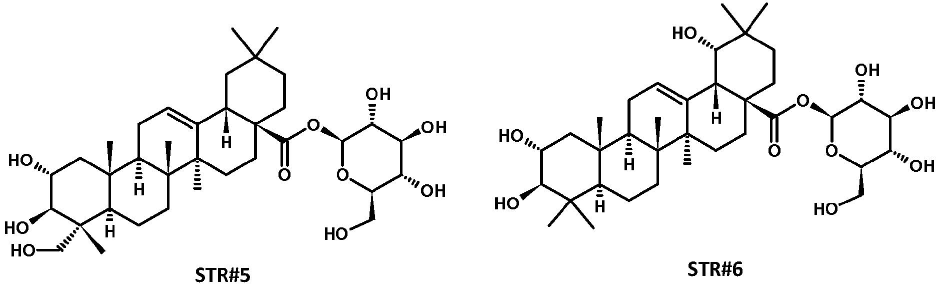

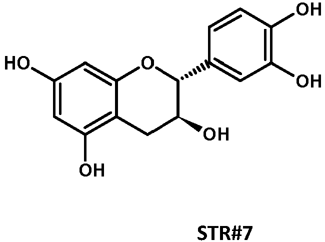

The solvent of step e) is ethyl acetate.

In another preferred embodiment, the invention discloses a composition comprising arjunoglucosides comprising Arjunoglucoside I represented by STR#4, Arjunoglucoside II represented by STR#5, wherein said composition further comprises Arjunic acid represented by STR#1, Arjunolic acid represented by STR#2, Arjungenin represented by STR#3, Arjunetin represented by STR#6, and catechin represented by STR#7, wherein said composition is obtained using steps a) to f) of the above process.

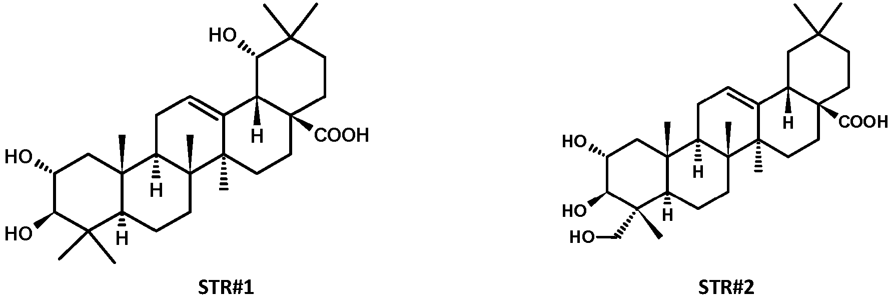

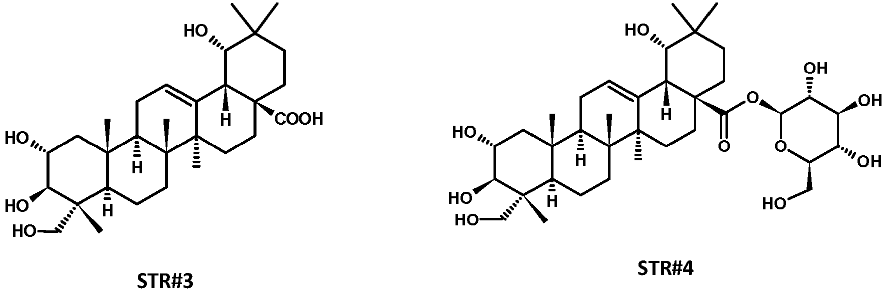

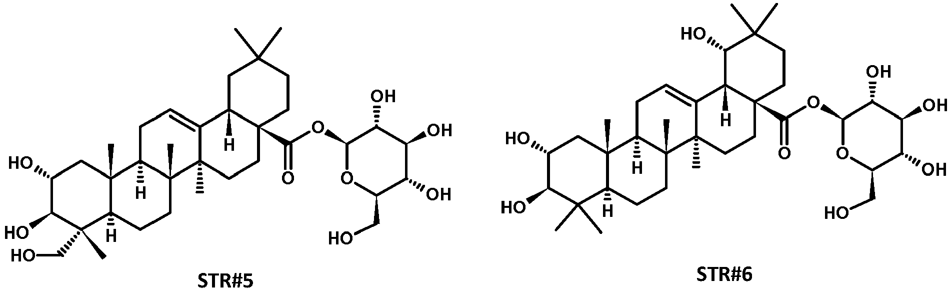

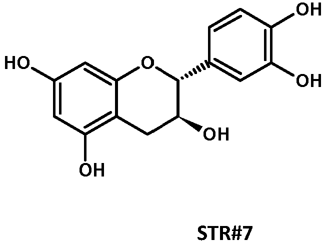

The specific examples included herein below illustrate the aforesaid most preferred embodiments of the present invention.

### Example 1: Process for isolating arjunoglucosides from the bark of Terminalia arjuna

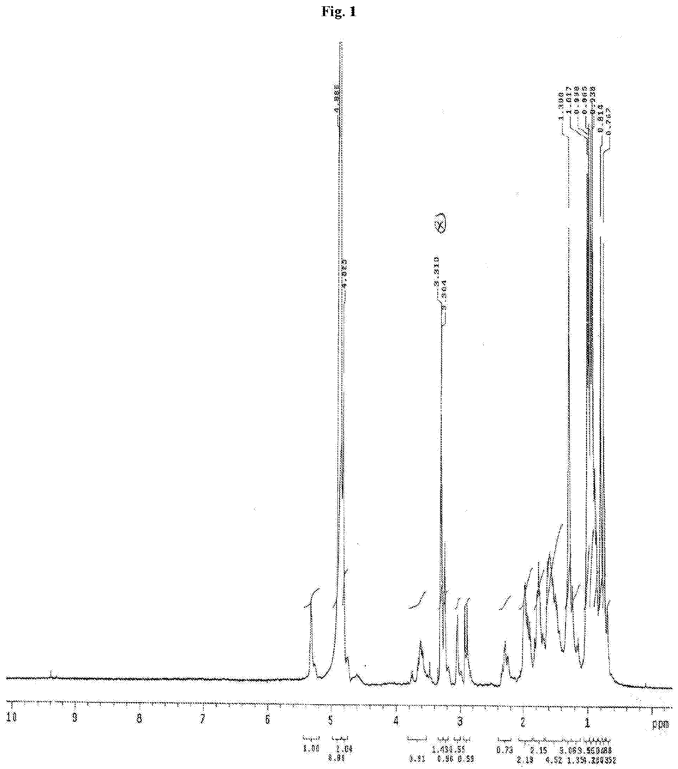

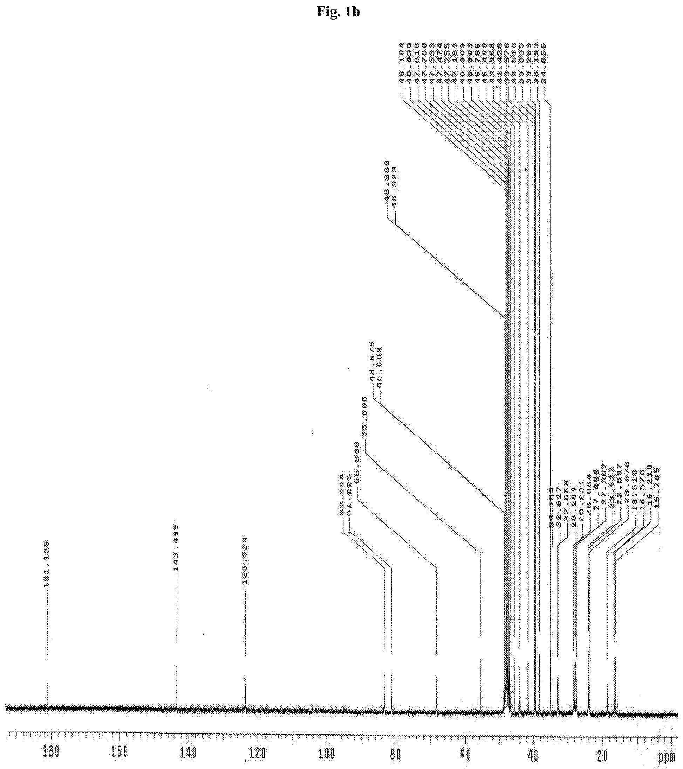

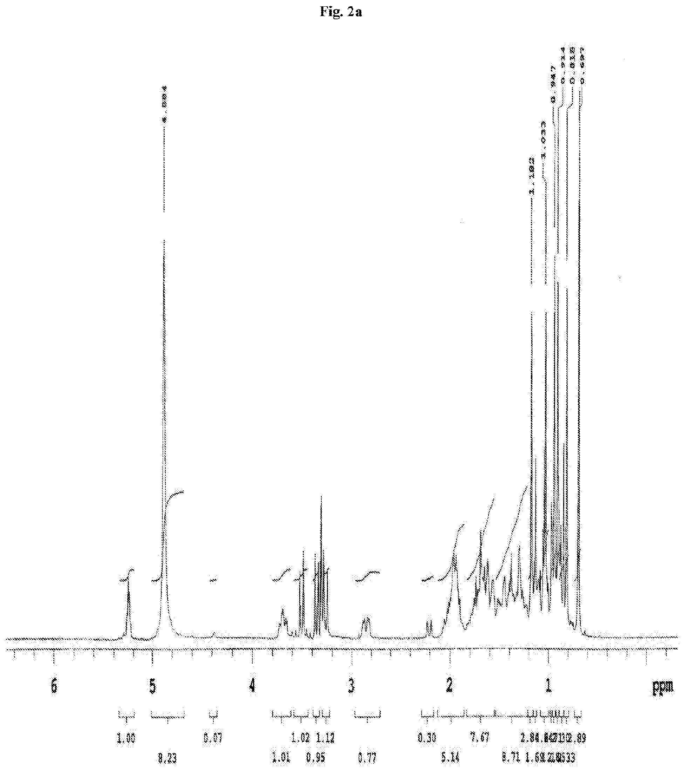

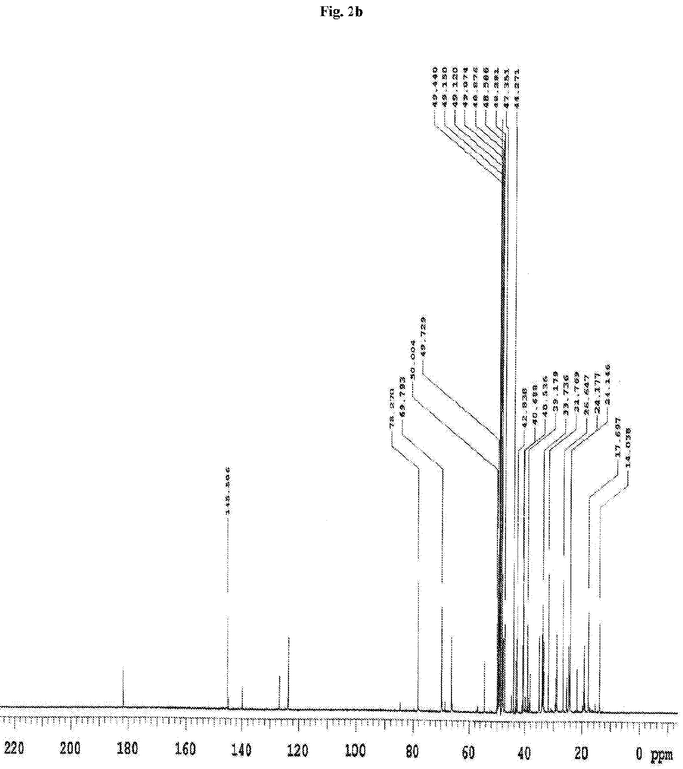

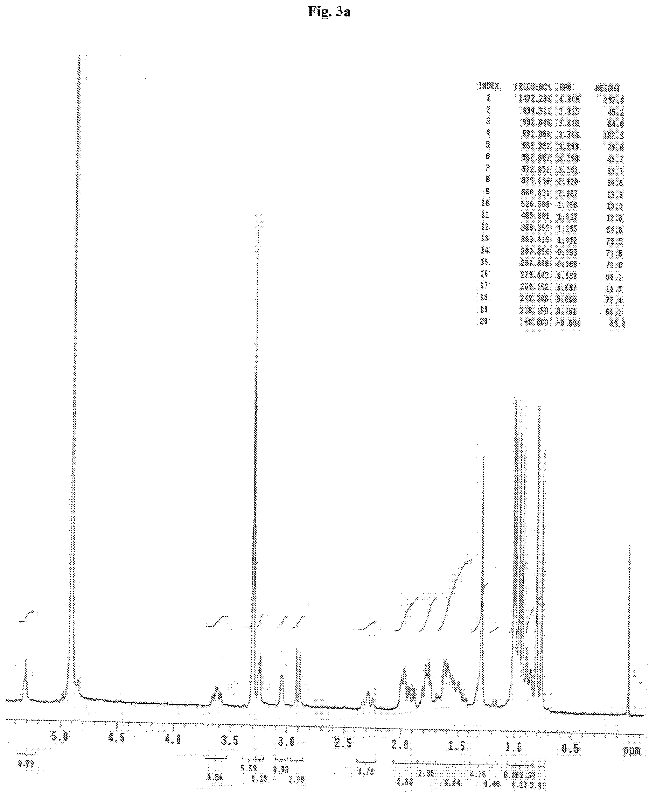

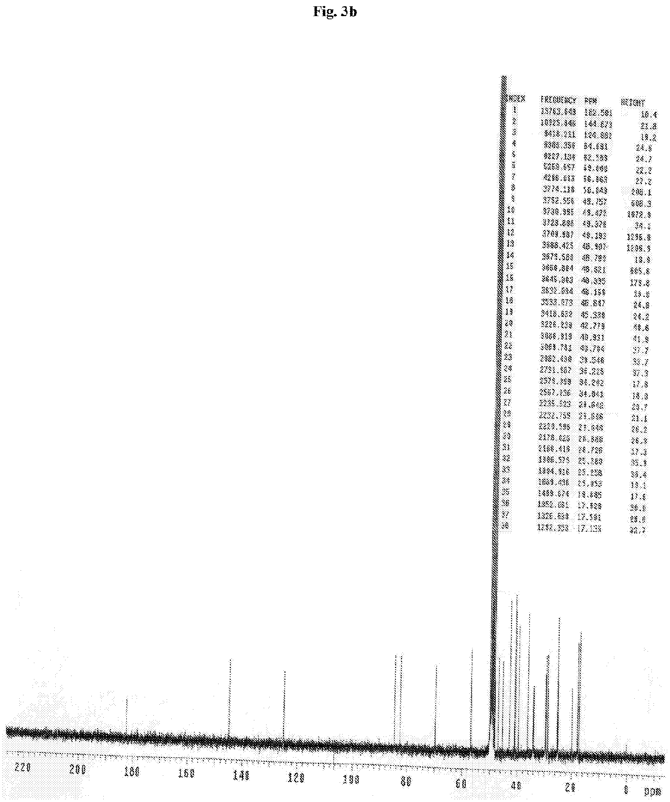

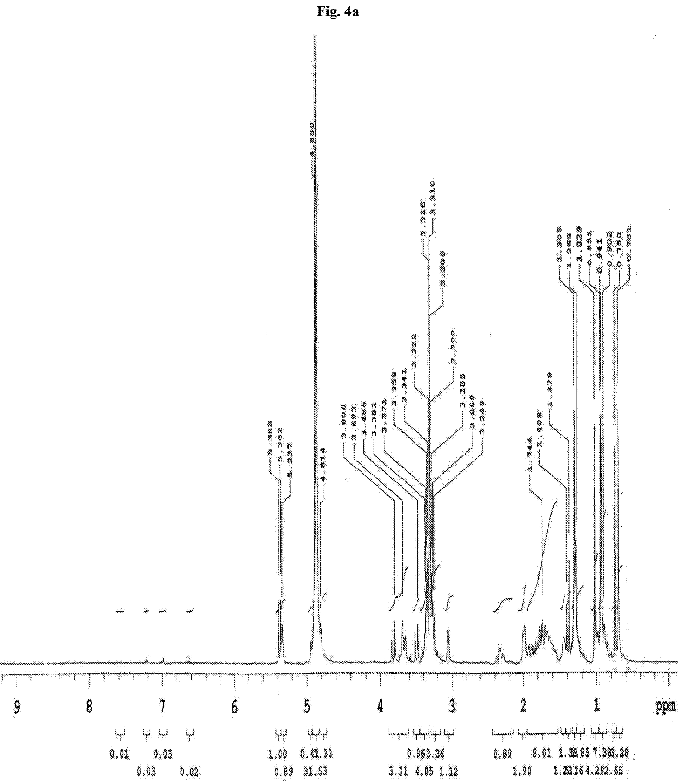

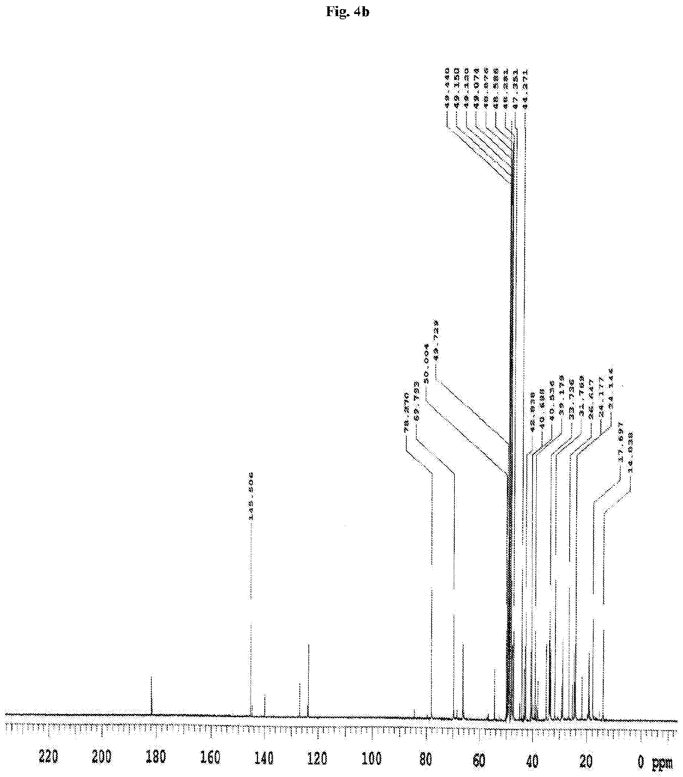

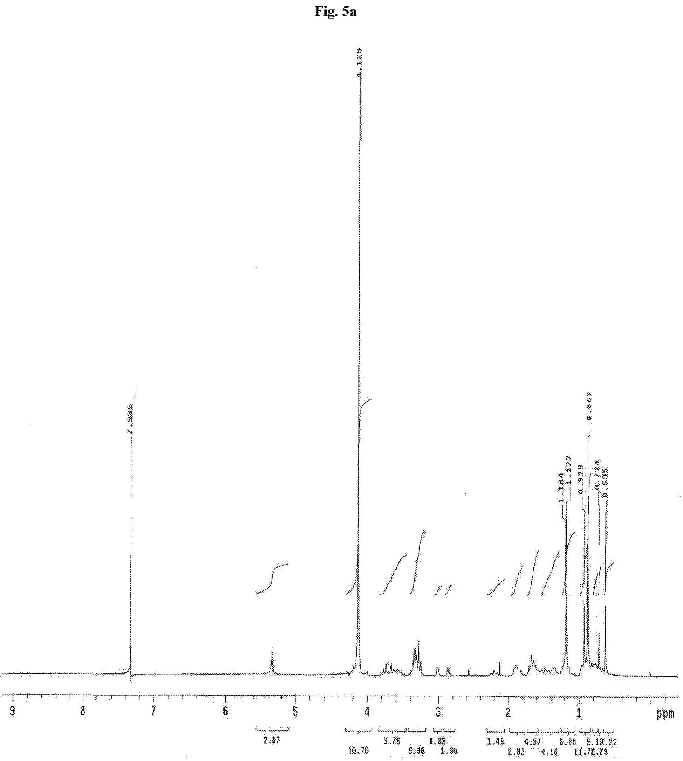

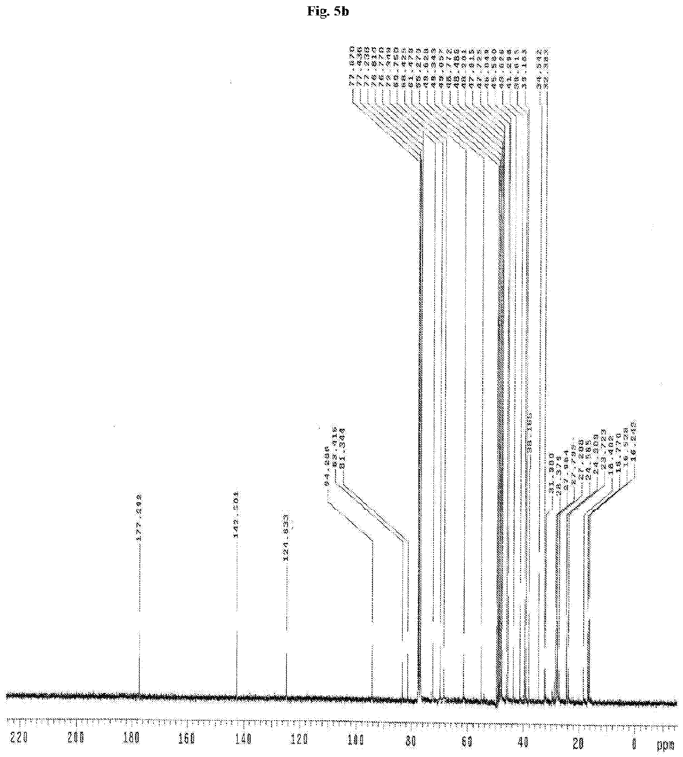

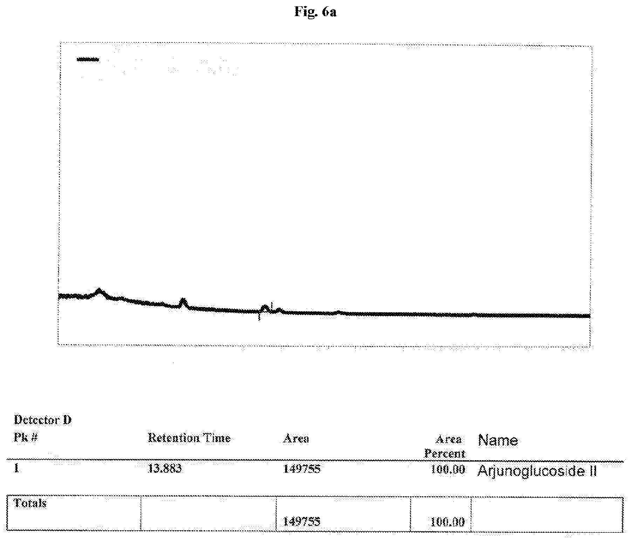

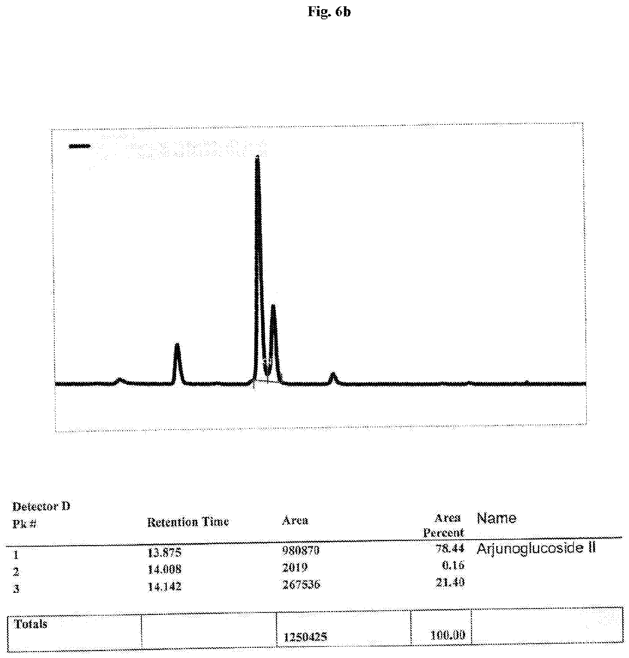

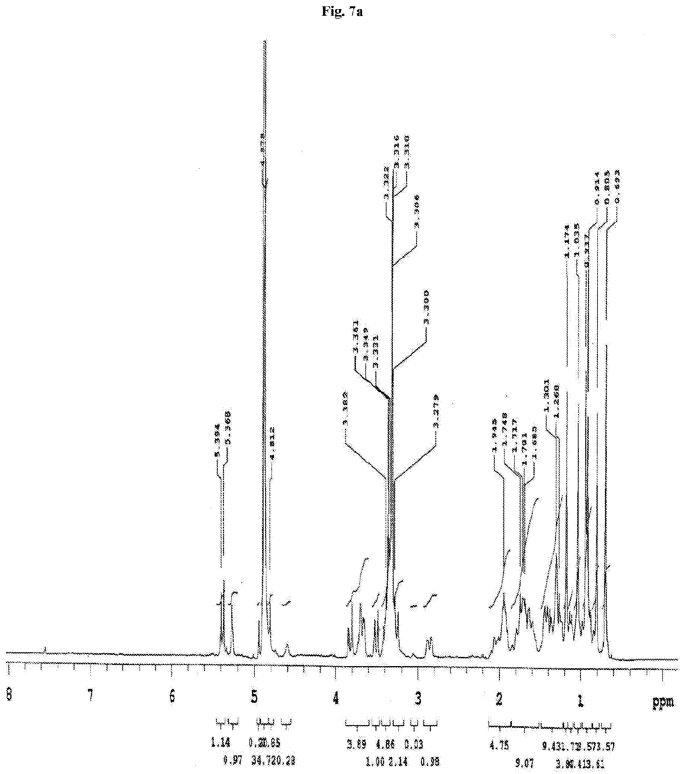

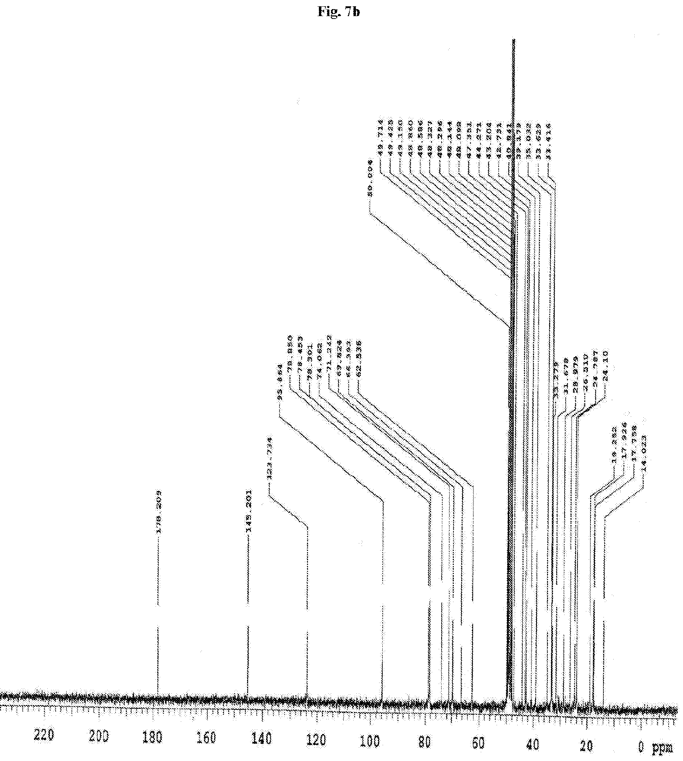

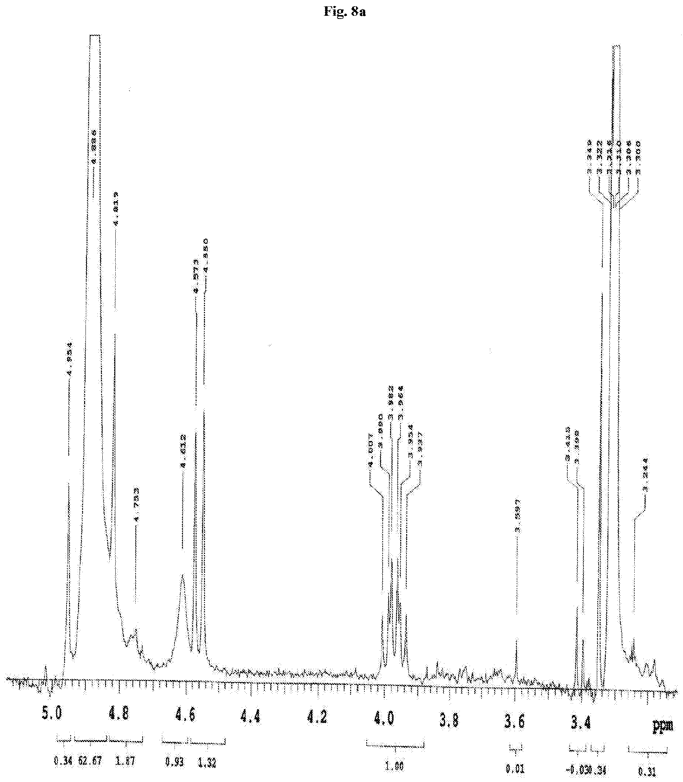

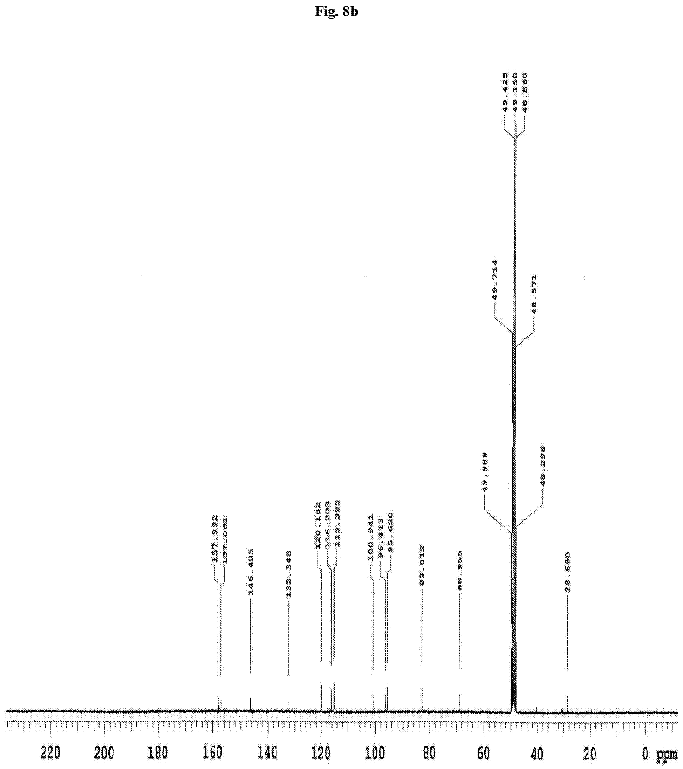

The present invention discloses a process for isolating arjunoglucosides from *Terminalia arjuna.* The process contains the following step of charging 2 kg of *Terminalia arjuna* bark powder into an extractor. Then added 3 volumes of methanol to *Terminalia arjuna* bark powder and refluxed for 3 hours at 65 to 70 °C. The solution was then filtered and concentrated under vacuum to get a brown coloured powder, Assay of arjunoglucoside-II by HPLC = 0.55% w/w, Yield approx. 500 g (25%). The powder was then dissolved into 5 volumes of de-mineralize water and stirred thoroughly for 1 hr at 50-55°C. The solution was then transferred to separating funnel and extracted with 2 volumes of ethyl acetate, 6 times, and the aqueous and organic layers were separated. The 6 ethyl acetate fractions were collected and concentrated under vacuum to obtain brown coloured powder, assay of arjunoglucoside-II by HPLC = 20-21% w/w, yield approx. 40 g. The powder was then loaded into a silica gel and eluted with chloroform: methanol. This fraction contains Arjunic acid, Arjunolic acid, Arjungenin, Arjunoglucoside-I & II, Arjunetin and catechin. The compounds were then identified using NMR as Arjunic acid (**Fig 1a and** **1b**), Arjunolic acid (**Fig 2a** **and** **2b**), Arjungenin (**Fig. 3a** **and** **3b**), Arjunoglucoside-I (**Fig. 4a** **and** **4b**), Arjunoglucoside-II (**Fig. 5a****,** **5b****,** **6a** **and** **6b**), Arjunetin (**Fig. 7a** **and** **7b**), and catechin (**Fig. 8a** **and** **8b**).

A composition containing atleast 3% arjunoglucosides comprising Arjunic acid, Arjunolic acid, Arjungenin, Arjunoglucoside-I, Arjunoglucoside-II, Arjunetin, and catechin was also formulated.

While the invention has been described with reference to a preferred embodiment, it is to be clearly understood by those skilled in the art that the invention is not limited thereto. Rather, the scope of the invention is to be interpreted only in conjunction with the appended claims.

## Claims

1. A process for the isolation of bioactive compounds from bark of *Terminalia arjuna,* said process comprising steps of:
(a) Charging *Terminalia arjuna* bark powder into an extractor,
(b) Adding 3 volumes of ethanol or methanol to *Terminalia arjuna* bark powder and refluxing for 3 hours at 65 to 70 °C,
(c) Filtering the ethanol or methanol extract of step b) and concentrating under vacuum to get a brown coloured powder,
(d) Dissolving the powder obtained from step c) into 5 volumes of de-mineralize water and stirring thoroughly for 1 hr at 50-55°C to obtain a solution,
(e) Transferring the solution from step d) to separating funnel and extracting with 2 volumes of solvent, 6 times, and separating the aqueous and organic layer, wherein the solvent is ethyl acetate,
(f) Collecting and combining the solvent fractions 1-6 from step e and concentrating under vacuum to obtain brown coloured powder,
(g) Loading the powder of step f) into a silica gel and eluting with chloroform: methanol,
(h) Identifying the compounds from step g) as Arjunic acid represented by STR#1, Arjunolic acid represented by STR#2, Arjungenin represented by STR#3, Arjunoglucoside-I represented by STR#4, Arjunoglucoside-II represented by STR#5, Arjunetin represented by STR#6, and catechin represented by STR#7.

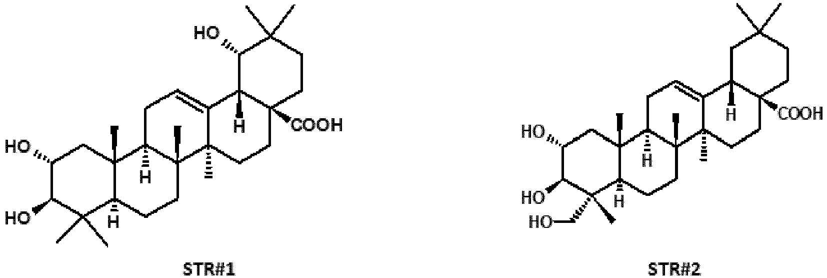

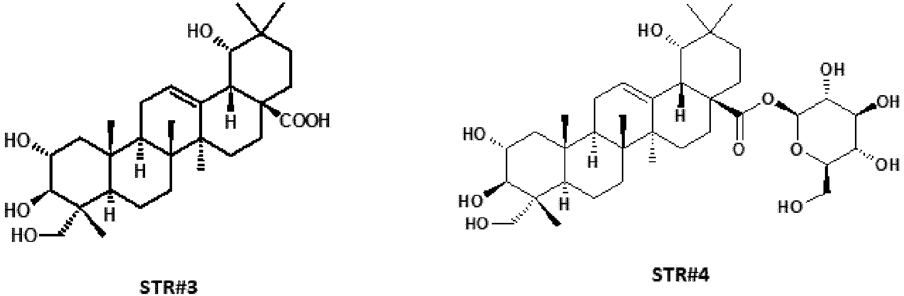

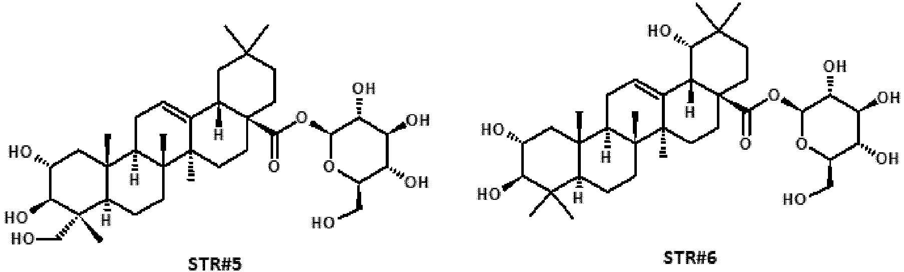

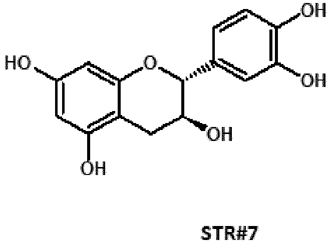

2. A composition comprising arjunoglucosides comprising Arjunoglucoside I represented by STR#4, Arjunoglucoside II represented by STR#5, wherein said composition further comprises Arjunic acid represented by STR#1, Arjunolic acid represented by STR#2, Arjungenin represented by STR#3, Arjunetin represented by STR#6, and catechin represented by STR#7, wherein said composition is obtained using a process according to steps a) to f) of claim 1.

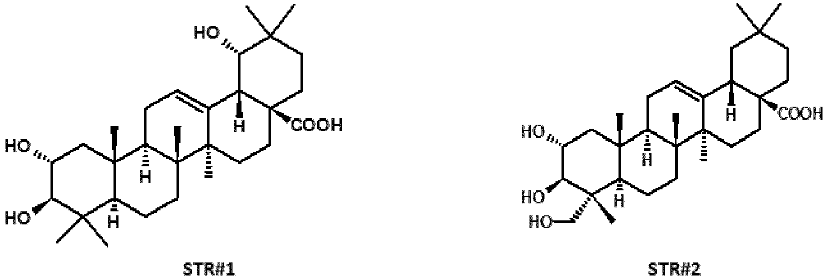

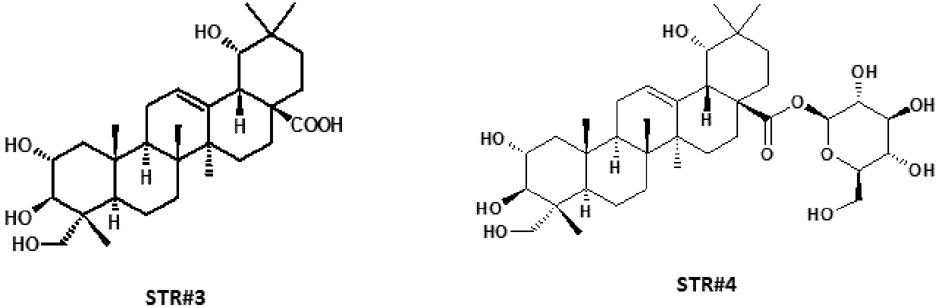

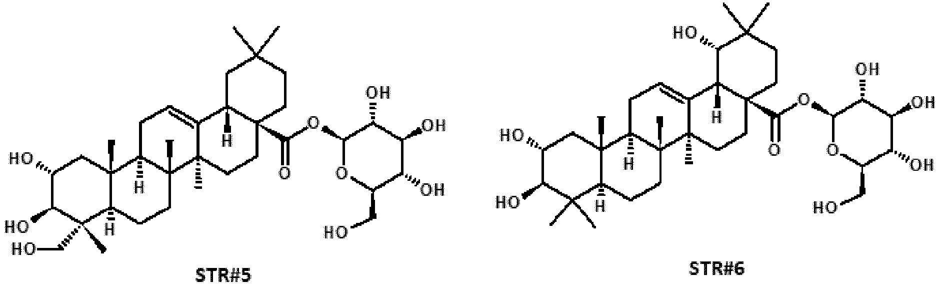

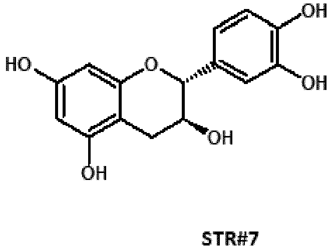

## Patentansprüche

1. Verfahren zur Isolierung bioaktiver Verbindungen aus der Rinde von *Terminalia arjuna*, wobei das Verfahren die folgenden Schritte umfasst:
(a) Einfüllen von *Terminalia arjuna*-Rindenpulver in einen Extraktor,
(b) Zugabe von 3 Volumenteilen Ethanol oder Methanol zu dem *Terminalia arjuna-*Rindenpulver und 3-stündiges Kochen am Rückfluss bei 65 bis 70 °C,
(c) Filtrieren des Ethanol- oder Methanolextrakts aus Schritt b) und Konzentrieren unter Vakuum, um ein braunes Pulver zu erhalten,
(d) Auflösen des aus Schritt c) erhaltenen Pulvers in 5 Volumenteilen entmineralisierten Wassers und gründliches Rühren für 1 Stunde bei 50-55 °C, um eine Lösung zu erhalten,
(e) Überführung der Lösung aus Schritt d) in einen Scheidetrichter und sechsmaliges Extrahieren mit 2 Volumenteilen Lösungsmittel sowie Trennen der wässrigen und organischen Schicht, wobei das Lösungsmittel Ethylacetat ist,
(f) Sammeln und Zusammenführen der Lösungsmittelfraktionen 1-6 aus Schritt e und Konzentrieren unter Vakuum, um ein braunes Pulver zu erhalten,
(g) Einbringen des Pulvers aus Schritt f) in ein Silicagel und Eluieren mit Chloroform : Methanol,
(h) Identifizierung der Verbindungen aus Schritt g) als Arjunsäure, dargestellt durch STR#1, Arjunolsäure, dargestellt durch STR#2, Arjungenin, dargestellt durch STR#3, Arjunoglucosid-I, dargestellt durch STR#4, Arjunoglucosid-II, dargestellt durch STR#5, Arjunetin, dargestellt durch STR#6, und Catechin, dargestellt durch STR#7.

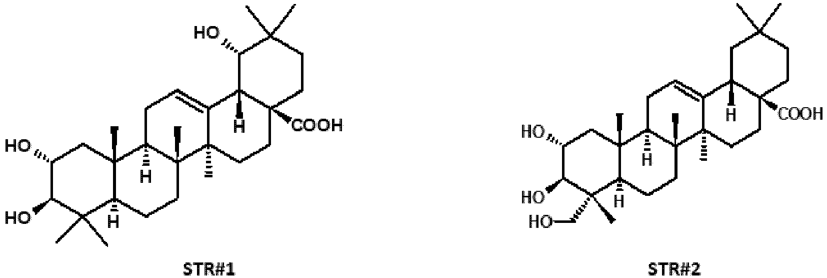

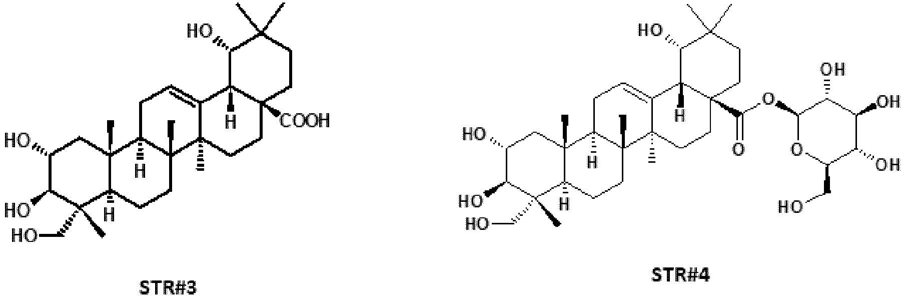

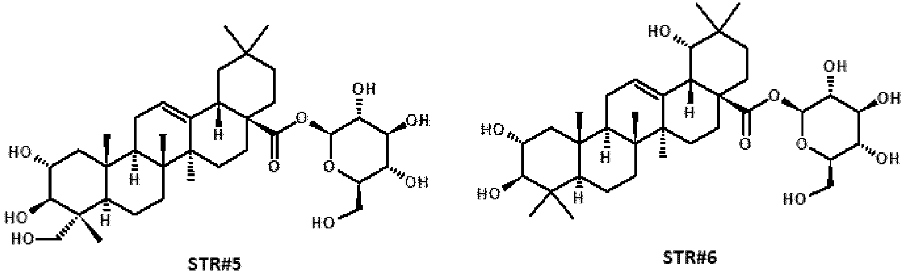

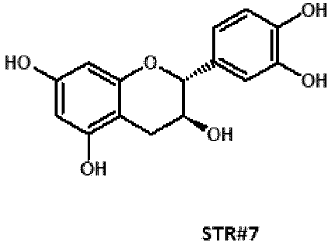

2. Eine Zusammensetzung, die Arjunoglucoside umfasst, umfassend Arjunoglucosid I, dargestellt durch STR#4, Arjunoglucosid II, dargestellt durch STR#5, wobei die Zusammensetzung weiterhin Arjuninsäure, dargestellt durch STR#1, Arjunolsäure, dargestellt durch STR#2, Arjungenin, dargestellt durch STR#3, Arjunetin, dargestellt durch STR#6, und Catechin, dargestellt durch STR#7, umfasst, wobei die Zusammensetzung unter Verwendung eines Verfahrens gemäß den Schritten a) bis f) des Anspruchs 1 erhalten wird.

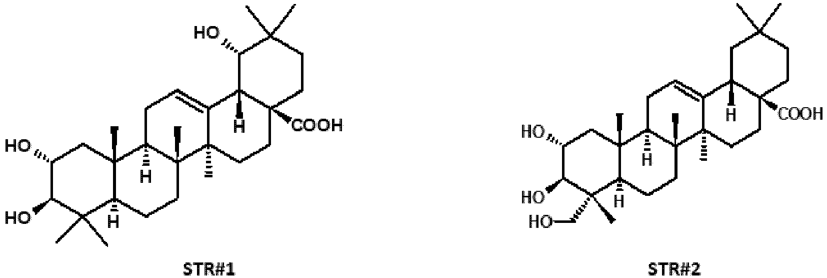

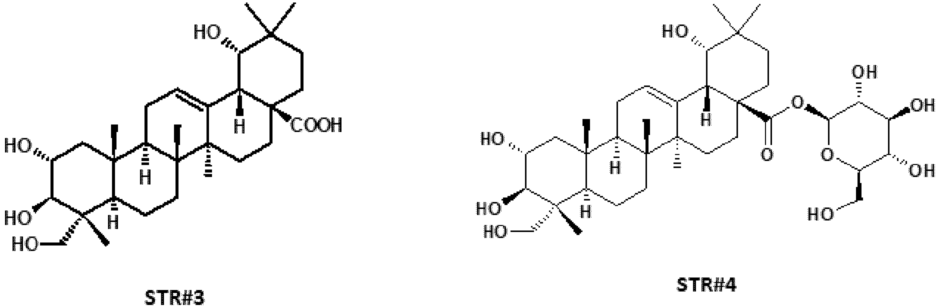

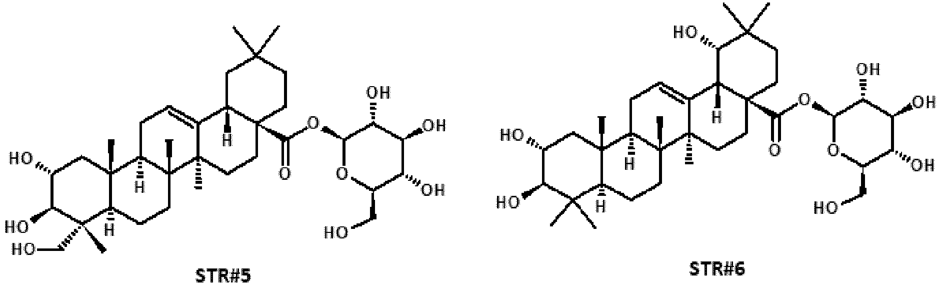

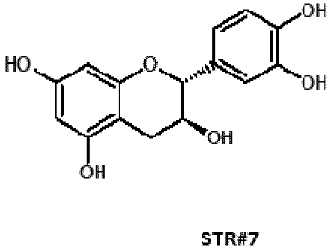

## Revendications

1. Procédé pour l'isolation de composés bioactifs à partir d'écorce de *Terminalia arjuna*, ledit procédé comprenant les étapes consistant à :
(a) charger de la poudre d'écorce de *Terminalia arjuna* dans un extracteur,
(b) ajouter 3 volumes d'éthanol ou de méthanol à la poudre d'écorce de *Terminalia arjuna* et maintenir à reflux pendant 3 heures à 65 à 70 °C,
(c) filtrer l'extrait d'éthanol ou de méthanol de l'étape b) et concentrer sous vide pour obtenir une poudre de couleur marron,
(d) dissoudre la poudre obtenue de l'étape c) dans 5 volumes d'eau déminéralisée et agiter minutieusement pendant 1 heure à 50 à 55 °C pour obtenir une solution,
(e) transférer la solution de l'étape d) vers un entonnoir de séparation et extraire avec 2 volumes de solvant, 6 fois, et séparer la couche aqueuse et organique, dans lequel le solvant est de l'acétate d'éthyle,
(f) recueillir et combiner les fractions de solvant 1 à 6 de l'étape e et concentrer sous vide pour obtenir une poudre de couleur marron,
(g) charger la poudre de l'étape f) dans un gel de silice et éluer avec du chloroforme/méthanol,
(h) identifier les composés de l'étape g) comme acide Arjunique représenté par STR#1, acide Arjunolique représenté par STR#2, Arjungénine représentée par STR#3, Arjunoglucoside-I représenté par STR#4, Arjunoglucoside-II représenté par STR#5, Arjunétine représentée par STR#6 et catéchine représentée par STR#7.

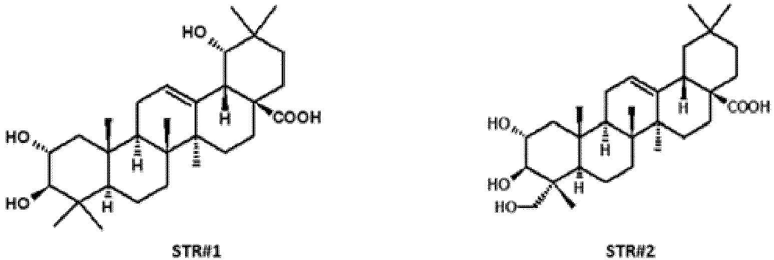

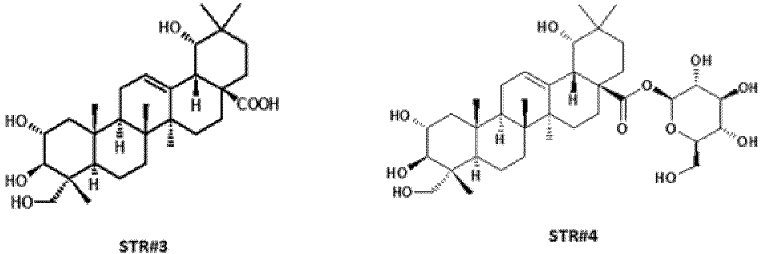

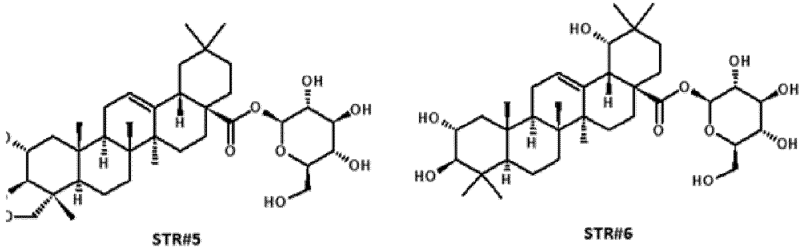

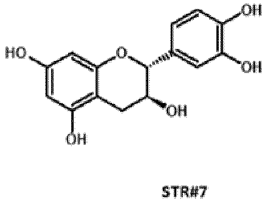

2. Composition comprenant des arjunoglucosides comprenant Arjunoglucoside-I représenté par STR#4, Arjunoglucoside-II représenté par STR#5, dans laquelle ladite composition comprend en outre de l'acide Arjunique représenté par STR#1, de l'acide Arjunolique représenté par STR#2, de l'Arjungénine représentée par STR#3, de l'Arjunétine représentée par STR#6 et de la catéchine représentée par STR#7, dans laquelle ladite composition est obtenue en utilisant un procédé selon les étapes a) à f) de la revendication 1.
